# EUROPEAN PATENT SPECIFICATION

(11) **EP 3 451 969 B1**
(45) Date of publication and mention of the grant of the patent: **22.11.2023**
(21) Application number: 17793469.2
(22) Date of filing: 05.05.2017
(51) Int. Cl.: A61F 2/14, A61F 9/01

(54) **CORNEAL IMPLANT SYSTEMS**
SYSTEME FÜR HORNHAUTIMPLANTATE
SYSTÈMES D'IMPLANT CORNÉEN

(30) Priority: 05.05.2016 US 201662332287 P; 31.08.2016 US 201662381952 P; 22.12.2016 US 201662438212 P; 23.01.2017 US 201762449114 P
(43) Date of publication of application: 13.03.2019
(73) Proprietor: Muller, David, Boston, MA 02111 (US)
(72) Inventor: MROCHEN, Michael, 8193 Eglisau (CH); STONE, Jennifer, Boston MA 02111 (US); MULLER, David, Boston MA 02111 (US)
(74) Representative: Mewburn Ellis LLP
(86) International application number: PCT/US2017/031339
(87) International publication number: WO 2017/193019

(56) References cited:
- WO-A2-2007/051951
- US-A1- 2009 018 532
- US-A1- 2010 241 224
- US-A1- 2010 241 224
- US-A1- 2014 264 980
- US-A1- 2014 264 980

## Description

The present disclosure relates generally to systems and methods for correcting vision, and more particularly, to systems and methods that employ implants to reshape the cornea in order to correct vision.

### BACKGROUND

A variety of eye disorders, such as myopia, hyperopia, astigmatism, and presbyopia, involve abnormal shaping of the cornea. This abnormal shaping prevents the cornea from properly focusing light onto the retina in the back of the eye (i.e., refractive error). A number of treatments attempt to reshape the cornea so that the light is properly focused. For instance, a common type of corrective treatment is LASIK (laser-assisted in situ keratomileusis), which employs a laser to reshape the cornea surgically.

### SUMMARY

According to aspects of the present disclosure, embodiments employ implants to reshape the cornea in order to correct vision. For instance, such embodiments may address the refractive errors associated with eye disorders such as myopia, hyperopia, astigmatism, and presbyopia. The implants may be formed from natural tissue, such as donor corneal tissue.

According to an example embodiment, a system for forming corneal implants includes a first cutting apparatus configured to cut a donor cornea and form a portion of corneal tissue. The donor cornea includes an anterior surface and a posterior surface. The first cutting apparatus is configured to cut the donor cornea along an axis extending between the anterior surface and the posterior surface. The system also includes a second cutting apparatus configured to form a plurality of lenticules from the portion of corneal tissue by making, to the portion of corneal tissue, a series of cuts transverse to the axis. Corneal tissue between consecutive cuts by the second cutting apparatus provides a corresponding lenticule to be shaped for a corneal implant. A distance between the consecutive cuts defines a thickness for the corresponding lenticule.

According to another example embodiment, a method for forming corneal implants includes providing a donor cornea including an anterior surface and a posterior surface. The method includes forming, with a first cutting apparatus, a portion of corneal tissue by cutting the donor cornea along an axis extending between the anterior surface and the posterior surface, the method includes forming, with a second cutting apparatus, a plurality of lenticules from the portion of corneal tissue by making, to the portion of corneal tissue, a series of cuts transverse to the axis. Corneal tissue between consecutive cuts by the second cutting apparatus provides a corresponding lenticule to be shaped for a corneal implant. A distance between the consecutive cuts defines a thickness for the corresponding lenticule. The method may further include freezing the portion of corneal tissue, wherein the second cutting apparatus includes a cryo-microtome configured to cut the frozen portion of corneal tissue.

In the example embodiments, the consecutive cuts made by the second cutting apparatus include a first cut made closest to the anterior surface of the donor cornea and a second cut made closest to the posterior surface of the donor cornea. The corresponding lenticule includes a first surface formed by the first cut and a second surface formed by the second cut. The second cutting apparatus may be further employed to shape at least one of the first surface or the second surface of the corresponding lenticule to form the corneal implant with a desired refractive profile.

### BRIEF DESCRIPTION OF THE DRAWINGS

FIG. 1A illustrates a view of an example implant formed from natural tissue according to aspects of the present disclosure.
FIG. 1B illustrates another view of the example implant of FIG. 1A.
FIG. 2 illustrates an example procedure employing an inlay implant formed from natural tissue, according to aspects of the present disclosure.
FIG. 3 illustrates an inlay implanted within corneal tissue, according to aspects of the present disclosure.
FIG. 4A illustrates an example procedure employing an onlay implant formed from natural tissue, according to aspects of the present disclosure.
FIG. 4B illustrates another example procedure employing an onlay implant formed from natural tissue, according to aspects of the present disclosure.
FIG. 4C illustrates yet another example procedure employing an onlay implant formed from natural tissue where one or more holes are formed in the Bowman's membrane, according to aspects of the present disclosure.
FIG. 5 illustrates an onlay implanted under a corneal epithelium, according to aspects of the present disclosure.
FIG. 6 illustrates an example implant for addressing surface irregularities, according to aspects of the present disclosure.
FIG. 7A illustrates a view of another example implant for addressing surface irregularities, according to aspects of the present disclosure.
FIG. 7B illustrates another view of the example implant of FIG. 7A.
FIG. 8 illustrates an example system for delaminating a donor cornea, according to aspects of the present disclosure.
FIG. 9 illustrates an example system for reshaping a lenticule, according to aspects of the present disclosure.
FIG. 10 illustrates an example approach for reshaping a lenticule, according to aspects of the present disclosure.
FIGS. 11A-B illustrate an example implantation of an implant employing a flap with a single attached portion.
FIGS. 12A-B illustrate an example implantation of an implant employing a flap with a plurality of attached portions arranged symmetrically about the flap, according to aspects of the present disclosure.
FIG. 13A illustrates another example system for forming a plurality of lenticules from a cornea, according to aspects of the present disclosure.
FIG. 13B illustrates an embodiment of the example system of FIG. 13A, according to aspects of the present disclosure.
FIG. 13C illustrates another embodiment of the example system of FIG. 13A, according to aspects of the present disclosure.
FIG. 14 illustrates an example implant with an edge (e.g., randomly serrated) designed to minimize glare and halo effect, according to aspects of the present disclosure.

While the invention is susceptible to various modifications and alternative forms, a specific embodiment thereof has been shown by way of example in the drawings and will herein be described in detail. It should be understood, however, that it is not intended to limit the invention to the particular forms disclosed, but on the contrary, the intention is to cover all modifications, equivalents, and alternatives falling within the spirit of the invention.

### DESCRIPTION

Example systems and methods employ implants to reshape the cornea in order to correct vision. For instance, such embodiments may address the refractive errors associated with eye disorders such as myopia, hyperopia, astigmatism, and presbyopia.

Example systems and methods employ implants that are formed from natural tissue. In particular, the implants may be formed from donor corneal tissue. For instance, the implants may be formed as allografts, i.e., tissue that is transplanted between members of the same species. Alternatively, the implants may be formed as xenografts, i.e., tissue that is transplanted between members of different species.

The methods and implants of the present disclosure exhibit significant improvements over prior attempts to correct vision utilizing implants. For example, some prior attempts to correct vision utilized implants made from synthetic materials; however, such implants made from synthetic materials did not work well for a variety of reasons (e.g., the irregularity of the collagen matrix of an eye, differences in the state of hydration of the synthetic material and the collagen matrix of an eye, lack of biocompatibility, etc.). The methods and implants of the present disclosure, which are made from natural tissue, overcome the deficiencies of such prior attempts. In particular, for example, the methods and implants of the present disclosure, which are made from natural tissue, exhibit greater biocompatibility with a patient's cornea, more closely match the index of refraction of the patient's cornea, can be maintained at a state of hydration that is required for implantation (e.g., a state of hydration that is similar to that of the implantation site), and ensures that sufficient gas and nutrients can be exchanged within the patient's cornea. Such advantages have not been achieved or successfully commercialized, at least in part, due to a lack of suitable methods and systems for manufacturing implants made from natural tissue.

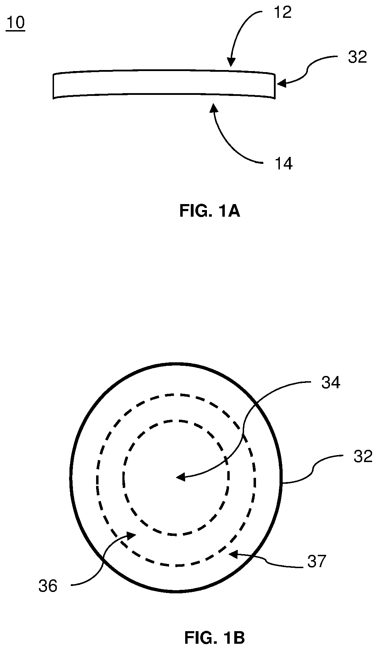

FIGS. 1A and 1B illustrate an example implant 10 according to aspects of the present disclosure. The implant 10 is formed from natural tissue or, more particularly, for example, a donor cornea. As shown in FIG. 1A, the implant 10 has a front (anterior) surface 12 corresponding to the anterior of the eye when implanted and a back (posterior) surface 14 corresponding to the posterior of the eye when implanted. While the example implant 10 illustrated in FIG. 1 has a front surface 12 and back surface 14 that form a meniscus shape, the implant 10 may have a plano-convex shape, a plano-concave shape, a bi-convex shape, or the like. Additionally, the front surface 12 and/or the back surface 14 may be spherical and/or aspherical.

To facilitate description of some aspects of the implant 10, FIG. 1B shows a top plan view of the implant 10 having a central region 34, a mid-peripheral region, 36, an outer peripheral region 37, and a peripheral edge 32. It should be understood that such regions 34, 36, 37 are intended as one non-limiting example and the implants 10 may have any number (i.e., one or more) of regions of any shape and size. Additionally, while the example implant 10 illustrated in FIGS. 1A and 1B has a circular perimeter shape defined by the peripheral edge 32, the implant 10 may have an oval shape, a polygonal shape, a nonpolygonal shape, or the like.

According to aspects of the present disclosure, the back surface 14 of the implant 10 may be shaped to have a surface profile that generally corresponds to a surface profile of an implantation site of a patient's cornea, and the front surface 12 of the implant 10 may be shaped to have a surface profile that provides a predetermined refractive correction. To achieve this, the implant 10 may be precisely custom formed for the patient receiving the implant 10.

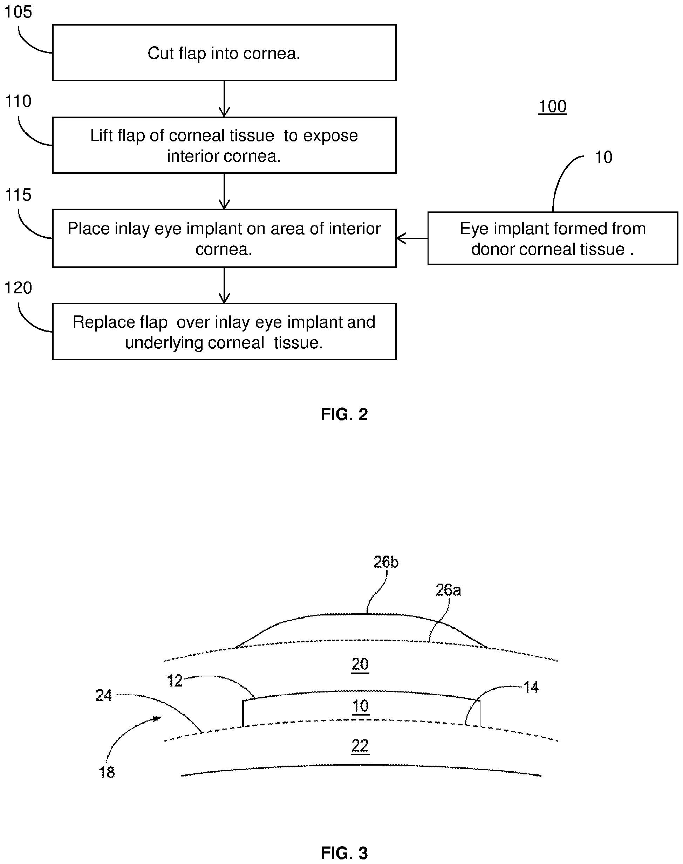

FIG. 2 illustrates an example procedure 100 for implantation of the implant 10 according to aspects of the present disclosure. In step 105, a flap is formed in a cornea 16. For example, a laser (e.g., a femtosecond laser), a mechanical keratome, other cutting mechanisms (e.g., a blade), etc., may be used to cut the flap. In some embodiments, the flap may be as thin as flaps that are cut for Sub-Bowman's Keratomileusis. The flap is sufficiently large to provide stability and ease of handling. In step 110, the flap of corneal tissue is lifted to expose the corneal interior 18. Thus, as a result of step 105 and step 110, an anterior portion 20 of the cornea 16 is separated from a posterior portion 22 of the cornea 16 to expose a stromal bed 24 upon which the implant 10 can be implanted.

In step 115, the implant 10 formed from donor corneal tissue is placed onto the stromal bed 24 at an implantation site in the exposed interior area 18 of the cornea 16 formed in step 105. The back surface 14 of the implant 10 is placed into contact with the bed 24 and may have a shape that corresponds to the shape of the bed 24 at the implantation site. In some cases, the back surface 14 of the implant 10 may have a non-flat surface curvature that generally corresponds to the non-flat curvature of the bed 24 at the implantation site. Alternatively, the back surface 14 of the implant 10 may be generally flat to correspond with a generally flat bed 24 at the implantation site. In some cases, the flap may have varying thicknesses to combine with the shapes of the implant 10 and the stromal bed 24 to produce the desired corneal shape.

According to some aspects, the implant 10 is implanted into the cornea 16 in a hydrated state. In some cases, the implant 10 may be transferred, via an insertion device (not shown), from a storage media containing the implant 10 prior to the procedure 100 to the implantation site. In other cases, the implant 10 may be transferred from a controlled environment directly and immediately to the implantation site. For example, the insertion device may be configured to maintain the implant 10 in the desired hydrated state. In step 120, the flap is replaced over the implant 10 and corneal interior 18. With the flap in place after step 120, the cornea 16 heals and seals the flap of corneal tissue to the rest of the cornea 16 (i.e., the anterior portion 20 seals to the posterior portion 22 to enclose the implant 10).

As shown in FIG. 3, after the procedure 100, the implant 10 is surgically inserted within the interior 18 of the cornea 16 with an anterior portion 20 of corneal tissue 16 disposed over the implant 10. Accordingly, in FIGS. 2-3, the implant 10 is implanted as an inlay implant because it is surgically implanted within the interior 18 of the cornea 16 (i.e., between the anterior portion 20 and a posterior portion 22 of the cornea 16). The implant 10 changes the shape of the cornea 16 as evidenced by a change in the anterior corneal surface 26a, 26b (e.g., in FIG. 3, the anterior corneal surface is shown as a dashed line 26a prior to the implantation and as a solid line 26b after implantation). This change in shape of the anterior corneal surface 26a, 26b results in corrective modification of the cornea 16, e.g., refractive correction, increase in depth of focus, etc. For example, the implant 10 may address the loss of near vision associated with presbyopia. To correct the effects of presbyopia, for instance, the implant 10 may be sized and positioned so that the change to the corneal shape improves near vision while having minimal effect on distance vision, which requires no correction. In general, however, the implants 10 may have any size or shape to produce the necessary desired correction. For instance, in some cases, the implant 10 may have a diameter of up to approximately 10 mm, but preferably not more than approximately 7 mm.

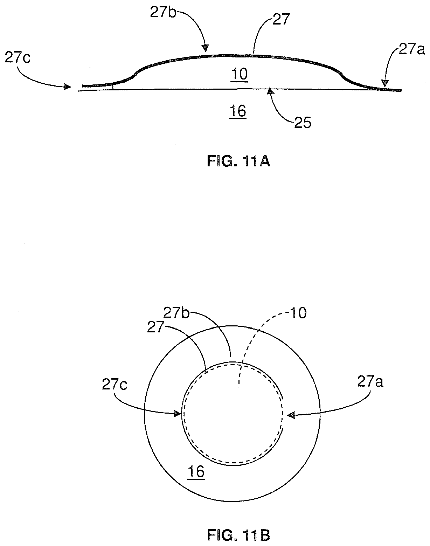

In the example procedure 100 illustrated in FIG. 2, the flap of corneal tissue is formed in step 105 and lifted in step 110 to expose the corneal interior 18, which receives the implant 10. The flap is then replaced in step 120 over the implant 10. FIGS. 11A-B illustrate an example implantation of the implant 10 employing a flap 27. The implant 10 has been received into a corneal bed 25 of the cornea 16 and the flap 27 has been placed over the implant 10. The flap 27 is attached to the rest of the cornea 16 at a single portion 27a. Meanwhile, the remaining edge 27b of the flap 27 has been incised from the cornea 16 and is not attached to the cornea 16. As such, when the flap 27 is lifted, the attached portion 27a effectively acts like a hinge. When the flap 27 is replaced after implant 10 is received by the bed 25, the flap 27 extends over the implant 10 with the attached portion 27a held down against the rest of the cornea 16. As shown in FIG. 11A, a section 27c of the edge 27b lies opposite the attached portion 27a. Because the opposing section 27c is not attached to the cornea 16, it does not lie flat against the cornea 16 in the same manner as the attached portion 27a. The flap 27 lies over the implant 10 asymmetrically and experiences asymmetric forces. If this asymmetry remains through healing and sealing of the flap 27 along the edge 27b, resulting asymmetric stresses on the anterior surface of the cornea 16 may induce an astigmatic shape for the cornea 16.

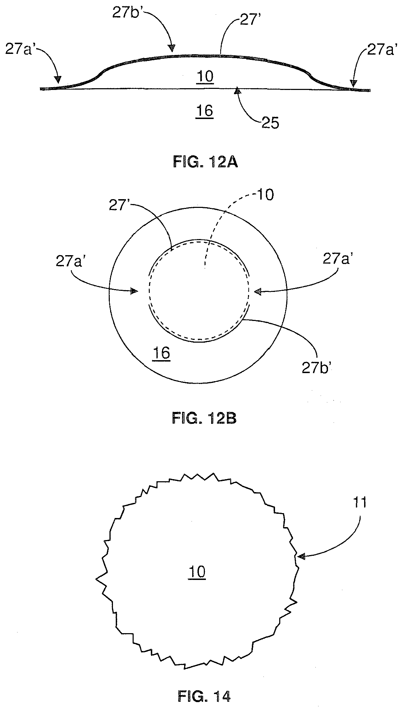

To reduce the likelihood of such asymmetric stresses, FIGS. 12A-B illustrate a approach for forming an alternative flap 27'. Rather than employing a single attached portion 27a as shown in FIGS. 11A-B, the flap 27' includes a plurality of attached portions 27a' that are arranged symmetrically about the flap 27'. As FIGS. 12A-B illustrate, the flap 27' includes two opposing attached portions 27a', which are both held down and lie flat against the cornea 16 in a similar manner. Unlike the example of FIGS. 11A-B, the flap 27 lies over the implant 10 symmetrically and experiences symmetric forces. With such symmetry during healing and sealing of the flap 27' at the unattached edge 27b', the anterior surface of the cornea 16 is less likely to experience asymmetric stresses that may induce an astigmatic shape for the cornea 16. In other words, the opposing attached portions 27a' produce equal and opposite stresses on the anterior surface of the cornea 16.

Although the flap 27' is attached at a plurality of areas, the implant 10 can be received under the flap 27' via the unattached edge 27b'. Although the flap 27' shown in FIGS. 12A-B includes two attached portions 27a', other embodiments may include more attached portions 27a'. For instance, the flap 27' may include four attached portions 27a' arranged symmetrically about the flap 27', i.e., at 0°, 90°, 180°, and 270°. Although the flap 27' may have a circular shape, other embodiments may employ other shapes, e.g., oval, rectangular, square, etc. In addition, the implant 10 may also have other shapes, dimensions, etc. In general, the use of symmetrically arranged attached portions may be used with pockets and other approaches for implanting onlay/inlay implants to reduce the likelihood of astigmatic effects.

As described above, the use of implants made from synthetic materials is accompanied by a variety of disadvantages. In particular, biologic and manufacturing limitations require synthetic implants to have a significant thickness at their periphery. Due to this thickness, the corneal tissue disposed over the synthetic implant experiences a draping effect at the periphery of the implant. This draping effect affects the shape of the cornea (e.g., at the anterior surface) beyond the periphery of the implant. In other words, if the synthetic implant has a particular diameter, the effect of the synthetic implant on the corneal shape is larger than the particular diameter, i.e., a larger effective zone. To achieve an effective zone for typical corrective reshaping, the synthetic implant must thus be limited to smaller diameters. For instance, the synthetic implant may be required to have a diameter of 4 mm or less. It is very difficult, however, to handle such smaller implants and to align and keep them in position.

The implants formed from natural tissue according to the present disclosure do not have the biologic and manufacturing limitations of synthetic implants and thus do not experience the same draping effect at their periphery. As such, natural implants of larger diameters, e.g., greater than 4 mm, may be employed to achieve corrective reshaping. For a given corneal shape change, the diameter of the natural implant can be larger than the synthetic implant. The implant procedure does not have to account for an effective zone that is significantly greater than the diameter of the natural implant. In some embodiments, the natural implant may have also a skirt that tapers to near-zero thickness at the periphery to minimize further any possible draping effect. The corneal tissue is supported by the skirt as the corneal tissue gradually rises and extends inwardly over the natural implant. With the skirt, the anterior surface is not affected beyond the periphery of the natural implant, so the corneal shape change corresponds more predictably to the size of the natural implant. With larger sizes, the natural implants do not suffer from the disadvantages of the synthetic implants described above.

While the implant 10 shown in FIG. 3 is employed as an inlay implant 10, it is understood that applying the implant 10 to the cornea 16 is not limited to the procedure 100 described above and that other procedures may be employed. For example, rather than forming a flap, a pocket having side walls with an opening may be formed (e.g., with a femtosecond laser or other cutting mechanism) to receive the implant 10. Stated more generally, the cornea 16 can be cut to separate the anterior portion 20 of the cornea 16 (e.g., the flap or an anterior section of a pocket) from the posterior portion 22 of the cornea 16, exposing the corneal interior 18 upon which the implant 10 can then be placed at an implantation site and subsequently covered by the anterior portion 20 of the cornea 16.

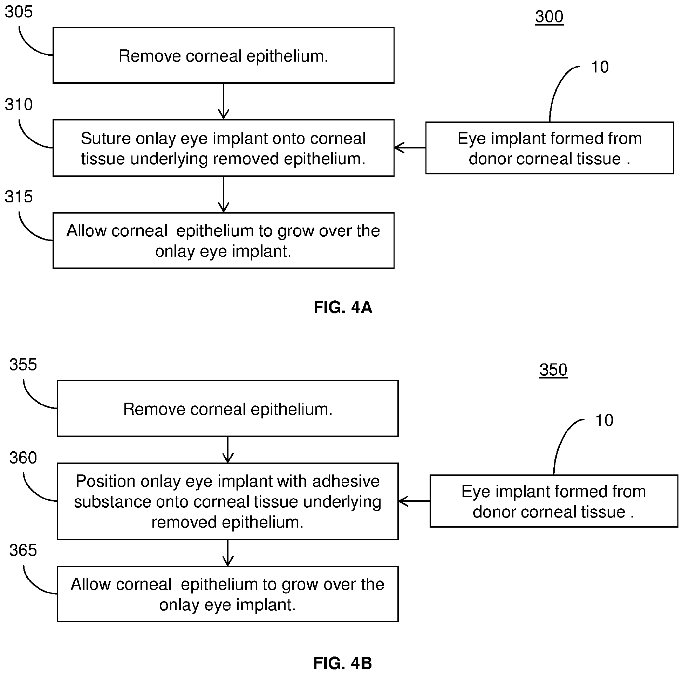

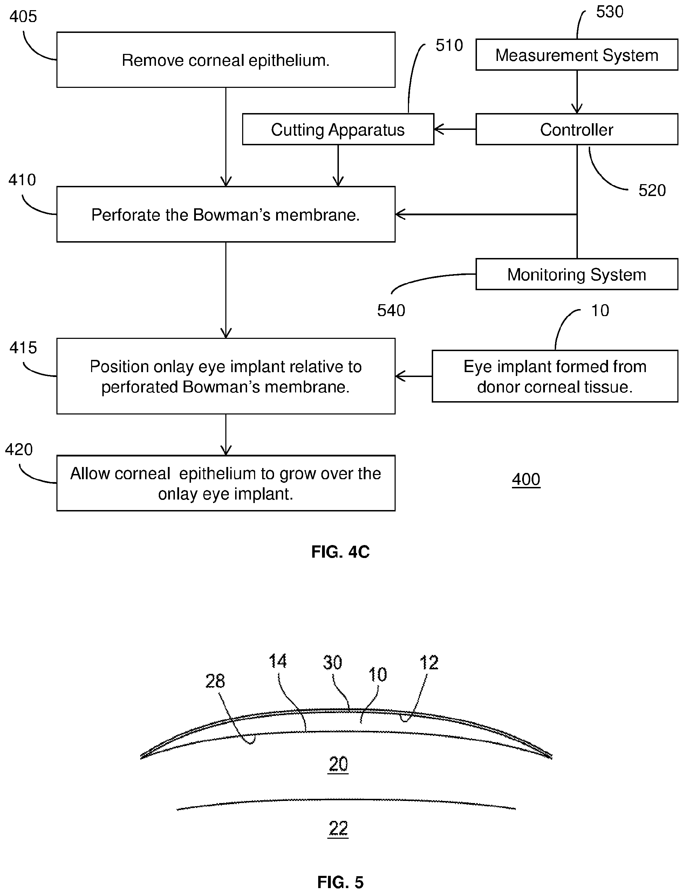

In other embodiments, the implant 10 may be employed as an onlay implant, where it is placed on an outer portion 28 of the cornea 16 just under the epithelium 30 so that the epithelium 30 can grow over the implant 10. For instance, in an example procedure 300 shown in FIG. 4A, at least a portion of the epithelium 30 is removed (e.g., scraped) from the cornea 16 in step 305 and the implant 10 is sutured over the outer portion 28 of the corneal tissue 16 in step 310 where the epithelium 30 is allowed to grow over the implant 10 in step 315.

Alternatively, in another example procedure 350 shown in FIG. 4B, at least a portion of the epithelium 30 is removed (e.g., scraped) from the cornea in step 355 and the implant 10 is stably positioned with an adhesive substance over the outer portion 28 of the corneal tissue 16 in step 360 where the epithelium 30 is allowed to grow over the implant 10 in step 365. The adhesive substance, for example, may be a synthetic, biocompatible hydrogel that creates a temporary, soft, and lubricious surface barrier over the implant 10, keeping the implant 10 in place for the growth of the epithelium 30. According to some aspects of the present disclosure, the adhesive substance can include a cross-linking agent. In one non-limiting example, the onlay implant 10 can be dipped into riboflavin to facilitate assist in visualizing placement of the implant 10 on the outer portion 28 of the cornea 16. After placement onto the outer portion 28, the cross-linking agent can be activated (e.g., via a photoactivating light) to hold the implant 10 to the outer portion 28 of the cornea 16.

Like the inlay implant, the onlay implant changes the shape of the cornea 16 and results in corrective modification of the cornea 16. Thus, the onlay implant may be applied to treat all refractive errors. As shown in FIG. 5, the corneal epithelium 30 grows over the onlay implant 10 which is implanted on the outer portion 28 of the corneal tissue 16. The epithelium 30 is generally about 50 micrometers (i.e., 5-6 cell layers) thick and generally regenerates when the cornea 16 is damaged or partially removed. To facilitate recovery after implantation, the shape of the implant 10 is configured to facilitate the advancement of the epithelium 30 smoothly over the implant 10 during regeneration. More particularly, the implant 10 can have a tapered profile at the outer peripheral region 37 such that the implant 10 becomes thinner from the mid-periphery region 36 towards the peripheral edge 32 of the implant 10. Formed from donor corneal tissue, the implant 10 advantageously promotes effective growth of the epithelium 30. In addition, the implant 10 provides the accuracy required to achieve the desired correction.

As described above, the onlay implant 10 is implanted on an outer portion 28 of the cornea 16 under the corneal epithelium 30. The Bowman's membrane is a smooth, acellular, nonregenerating layer, located between the epithelium and the stroma in the cornea of the eye. It is the outermost layer just below the epithelium. According to some aspects, the onlay implant 10 may be implanted between the Bowman's membrane and the epithelium 30. According to additional and/or alternative aspects, the onlay implant 10 may be implanted between one or more cell layers of the epithelium 30. According to still other additional and/or alternative aspects, the onlay implant 10 may be implanted such that a minor portion penetrates the Bowman's membrane and/or the stroma so long as a major portion of the onlay implant 10 is located on or above the Bowman's membrane and under the outermost layer of the epithelium 30.

According to one approach, a slight relief (e.g., cavity) is formed in the Bowman's layer to facilitate positioning of the onlay implant 10 and to help keep the onlay implant 10 in position during healing. This approach can be employed to lower the edges of the onlay implant 10, so that epithelial under growth is prevented and the epithelium 30 can grow more easily over the onlay implant 10.

During procedures for implanting the implant 10, the cornea may be contained in an environment that allows the cornea 16 to maintain a state that replicates the state of a cornea during conventional LASIK procedure, for instance.

According to another approach, an ultraviolet femtosecond laser is employed to create a separation between the Bowman's membrane and the epithelium for receiving an implant and reshaping the cornea. Although infrared femtosecond lasers may be employed to form pockets in the stroma, such lasers are not suitable for forming the separation between the Bowman's membrane and the epithelium. The epithelium is approximately 50 µm in thickness and the Bowman's membrane is approximately 8 to 10 µm in thickness. An infrared femtosecond laser can generate a plasma plume of up to 10 to 20 µm. As such, if the focus of an infrared femtosecond laser were directed at the surface of the Bowman's membrane, the Bowman's membrane would be destroyed. If, on the other hand, the focus of an infrared femtosecond laser were directed high enough into the epithelium to prevent damage to the Bowman's membrane, epithelium cells would remain as an undesirable result.

Meanwhile, an ultraviolet femtosecond laser generates a plasma plume that is only approximately 2 to 3 µm. As such, with an ultraviolet femtosecond laser, only the top few microns of the Bowman's membrane can be removed to create the desired separation between the Bowman's membrane and the epithelium. In some embodiments, monitoring technologies, such as optical coherence tomography (OCT), may be employed to locate the Bowman's membrane more accurately and guide the focus of the laser to the appropriate location. Unlike the present approach which specifically removes (e.g., ablates) a very thin layer of the Bowman's membrane with technology that has not been heretofore contemplated for such a use, other approaches employ an infrared femtosecond laser to create a separation at the cleavage plane and are not directed to the Bowman's membrane.

According to yet another approach, a laser is employed to create a pocket within the epithelium, close to the Bowman's membrane. A cutting instrument can then be inserted into the pocket to clean/separate the Bowman's membrane layer from the residual epithelium layers. The pocket is thus prepared to receive an implant.

According to some aspects of the present disclosure, the implant 10 (i.e., as an inlay or as an onlay) can be shaped to accommodate a single zone of power for vision correction. As a non-limiting example, the implant 10 can be shaped primarily to accommodate near-vision. As another non-limiting example, the implant 10 can be shaped to accommodate mid-vision or far-vision. According to other aspects of the present disclosure, the implant 10 can be shaped to provide multi-focality, e.g., accommodate more than one zone of different power. For example, the implant 10 can include a plurality of different portions that are each shaped to accommodate a different zone of power. While the implant 10 illustrated in FIG. 1 is described as having a central region 34, a mid-peripheral region 36, and an outer peripheral region 37, it should be understood that the implant 10 can have any other number of regions, each having a different power. As one non-limiting example, the central region 34 of the implant 10 may be shaped to accommodate near-vision, the mid-peripheral region 36 of the implant 10 may be shaped to accommodate mid-vision, and/or the outer peripheral region 37 of the implant 10 may be shaped to accommodate far-vision.

In general, a single implant 10 may be shaped with varying contours to induce regional corneal shape changes that treat a combination of disorders. For instance, the implant 10 can be shaped to treat presbyopia in combination with myopia or hyperopia. Indeed, most people requiring correction for presbyopia are also ametropic. For a patient with presbyopia and myopia, the implant 10 may have a shape is raised at the periphery with a cavity/hole in the center, allowing for some steepness in the center of the cornea. Meanwhile, for a patient with presbyopia and hyperopia, the implant 10 may have a shape that is convex at the periphery but has a raised center portion.

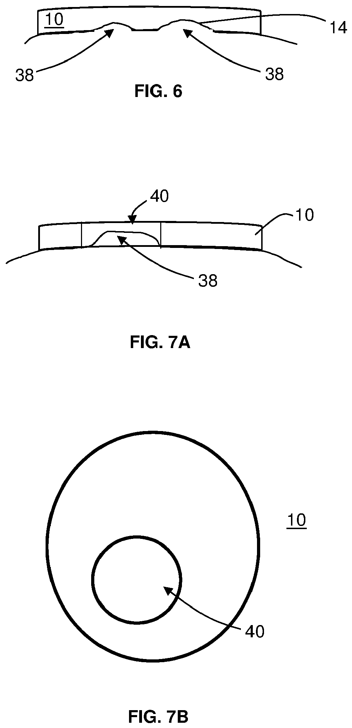

In some cases, patients with ectasia or keratoconus, for example, have corneal surface irregularities. Because their corneas 16 are typically thinner than normal, ablation techniques cannot be employed to smooth the shape of the corneas 16 to a more regular shape. To address this problem, a custom implant 10 (i.e., an inlay or an onlay) may be formed to have a shape that is generally the inverse of the surface irregularity and thus compensates for the surface irregularity. The implant 10 may be formed to have a front surface 12 that generally reproduces the back surface 14 curvature. For example, the implant 10 may be relatively thinner over areas of the cornea 16 that are relatively higher (i.e., extend outwardly), and vice versa. A non-limiting example of an onlay implant 10 that having a back surface 14 that is the inverse of the surface irregularities 38 of the outer portion 28 of the cornea 16 is illustrated in FIG. 6. The implant 10 may even have an aperture 40 that is positioned over steep and high portions of the cornea 16. For example, FIGS. 7A-7B illustrate a non-limiting example of an onlay implant 10 having an aperture 40 over a steep and high portion 42 of the outer portion 28 of the cornea 16. The implant 10 may be implanted as an inlay or an onlay according to the techniques described above.

Example embodiments may compensate for the defocus shift of the eye's optics associated with the transformation of the corneal shape caused by an implant. In particular, the example embodiments may consider the equations presented by Smith et al., "Effect of defocus on on-axis wave aberration of a centered optical system," Journal of the Optical Society of America A: Optics, Image Science, and Vision (2006), Vol. 23, Issue 11, pp. 2686-2689, the contents of which are incorporated entirely herein by reference.

After corneal implantation, the patient may observe a glare and halo caused by the edge of the implant. In particular, a round edge of the implant may cause a diffractive (or other optical) effect that produces a round image on the retina. To address a similar problem with LASIK procedure, a very large optical zone may be employed so that the halo occurs outside the visual zone. Such a solution, however, cannot be employed with the implant.

Accordingly, to minimize the glare and halo effect from an implant, implants according to the present disclosure may be shaped to have a randomly serrated edge rather than a smooth round edge. The serrations in the edge may include differently sized peaks, valleys, etc. As such, the diffractive (or other optical) effect caused by the edge is diffused and does not create a halo on the retina. Alternatively, to minimize the glare and halo effect from an implant, the implant may include a predefined edge that is structured to produce constructive and destructive interference and to manipulate the focus and depth of field. FIG. 14 illustrates, as a simplified example, an implant 10 with an edge 11 (e.g., randomly serrated) designed to minimize glare and halo effect.

The implants can be precisely produced according to patient specific conditions. For instance, the implants of the present disclosure can be manufactured to have a shape that generally corresponds to a shape of an implantation site of the patient's cornea, provides a predetermined amount of refractive correction, and/or addresses corneal irregularities. Approaches for producing eye implants from donor corneal tissue are described, for instance, in U.S. Patent Application Publication No. 2014/0264980, filed January 10, 2014, and U.S. Patent Application Publication No. 2017/0027754, filed February 28, 2016.

To produce eye implants from a donor cornea, the donor cornea may be delaminated to form a plurality of laminar sheets. A plurality of lenticules can then be cut from the laminar sheets and shaped for use as eye implants. The laminar sheets, for instance, may have a thickness of approximately 10 µm to approximately 50 µm; however, it should be understood that the laminar sheets can have other thicknesses. As used herein, the term "laminar sheet" may refer to a sheet that corresponds to a layer of the cornea defined by the cornea's lamellar structure.

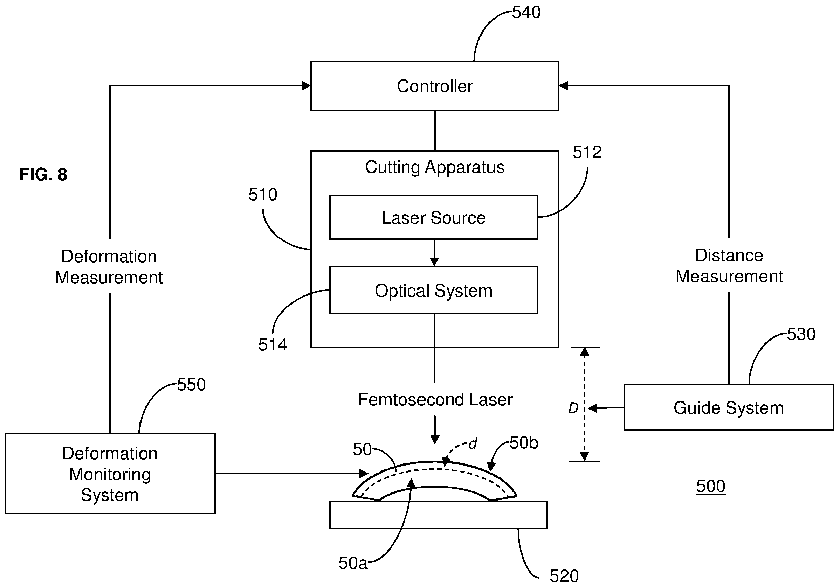

FIG. 8 illustrates an example delaminating system 500 for delaminating a donor cornea 50. The example system 500 includes a delaminating cutting apparatus 510. The cutting apparatus 510 includes a light source 512 that generates radiation for dissecting the tissue 50a of the donor cornea 50 via photodisruption, photothermal cavitation, laser spallation, etc. The cutting apparatus 510 also includes an optical system 514 that can focus the radiation on the tissue 50a and cut the cornea 50 at a desired depth d below the anterior surface 50b of the cornea 50. In some embodiments, the cutting apparatus 510 may generate a femtosecond laser to cut the cornea 50. In addition, the example system 500 includes a holding device 520 that mechanically positions the donor cornea 50 to receive the radiation from the cutting apparatus 510.

The structure of underlying collagen imposes a concave shape on the cornea 50. If the cornea 50 is compressed and/or experiences other stresses, the cornea 50 may deform from this concave shape. For instance, in some systems that incise the cornea (e.g., ophthalmic surgical systems), an aplanation device is applied to the anterior surface of the cornea to position the cornea relative to the cutting device. The aplanation device, for instance, may be formed from glass. When the aplanation device is applied, the cornea 50 typically flattens. If a cornea is flattened by an aplanation device and laminar sheets are obtained by cutting the flattened cornea at the predetermined depth d, the resulting laminar sheets might not have the desired uniform thicknesses due to the structure of the underlying collagen. In general, deformation of the cornea 50 may prevent the cutting apparatus 510 from accurately cutting the cornea 50 at the predetermined depth d.

Advantageously, the holding device 520 in FIG. 8 engages the cornea 50 without substantially deforming the cornea 50. As such, the cutting apparatus 510 can cut laminar sheets of more uniform thickness from the cornea 50.

When an aplanation device is employed to flatten the cornea, the aplanation device can be used as a reference point and the cornea can be cut at a predetermined depth from the aplanation device to obtain laminar sheets of desired thickness. In addition to introducing deformation that may affect the accuracy of cutting laminar sheets of desired thickness, however, an aplanation device may disadvantageously prevent the entire cornea from being used to form the laminar sheets. In other words, the aplanation device may not allow laminar sheets to be cut from sections of the cornea 50 extending fully from limbus to limbus.

In contrast, the example system 500 does not employ an aplanation device, so the example system 500 can use more of the cornea 50 and produce eye implants from the cornea 50 more efficiently. In particular, the example system 500 includes a guide system 530 that can guide the cutting apparatus 510 to cut laminar sheets from limbus to limbus of the cornea 50.

With the holding device 520 maintaining the cornea 50 at a fixed distance relative to the cutting apparatus 510, the guide system 530 can measure a distance D from the cutting apparatus 510 to the cornea 50, e.g., the surface 50b. The guide system 530 can communicate this distance measurement to a controller 540 that controls aspects of the cutting apparatus 510. The distance measurement provides the cutting apparatus 510 with the necessary reference to align and focus the femtosecond laser at a desired depth within the corneal tissue 50a. The femtosecond laser spot can be scanned over the cornea 50 to focus on points at the desired depth within the corneal tissue 50a to cut a laminar sheet extending fully from limbus to limbus. Unlike other systems, an aplanation device is not required in the example system 500 to provide a reference to determine focus depth for the femtosecond laser. Advantageously, by cutting the cornea 50 in this way, the concave contour of the posterior surface of the laminar sheet is also known/controlled for subsequent processing.

Although the optical system 520 can move the femtosecond laser over a stationary cornea 50, the holding device 520 in alternative embodiments can move the cornea 50 relative to a stationary irradiation system 520 (similar, for instance, to the operation of a goniometer stage), while the femtosecond laser cuts the cornea 50. In general, relative movement between the cornea 50 and the cutting apparatus 510 is guided by the guide system 530 to align and focus the femtosecond laser to the appropriate positions in the corneal tissue 50a to delaminate the cornea 50 at the predetermined depth d.

The example system 500 may also include a deformation monitoring system 550 that can measure any deformation of the cornea 50 that may occur while the cutting apparatus 510 cuts the cornea 50. For instance, the monitoring system 540 may employ second harmonic imaging, autofluorescence imaging, Brillouin scattering measurement, and/or x-ray scattering measurement before and during the cutting process to determine any deformation. If necessary, the optical system 514 can be adjusted to account for the deformation and allow the cornea 50 to be cut more accurately at the predetermined depth d. For instance, the focal depth of a femtosecond laser generated by the illumination system 510 can be adjusted to cut the cornea 50 with the desired thickness. The deformation monitoring system 550 can communicate the deformation measurement to the controller 540 which controls aspects of the cutting apparatus 510.

According to an example embodiment, the cutting apparatus 510 may apply a femtosecond laser also to induce gas bubbles at particular locations in the tissue 50a. The gas bubbles provide markers that can be monitored with the monitoring system 540. Movement of the gas bubbles can be measured to determine a deformation of the cornea 50.

Although the example system 500 may employ the holding device 520 which does not deform the cornea 50, aspects of the deformation monitoring system 540 may be employed in systems that employ an aplanation device. Such systems can then make necessary corrections to cut the cornea accurately at desired depths even with the deformation induced by the aplanation device.

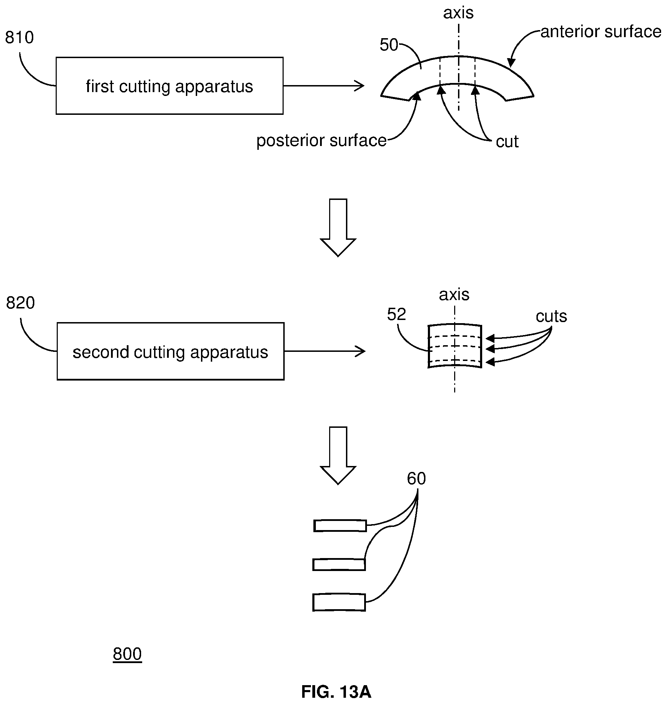

FIG. 13A illustrates an example system 800 for forming a plurality of lenticules 60 from a donor cornea 50. The system 800 includes a first cutting apparatus 810 that can cut the donor cornea 50 to form a portion 52 of corneal tissue. The donor cornea 50 includes an anterior surface and a posterior surface. The first cutting apparatus 810 can cut the donor cornea 50 along an axis extending between the anterior surface and the posterior surface as illustrated in FIG. 13A.

After operation of the first cutting apparatus 810, the portion 52 of corneal tissue, for instance, may be substantially cylindrical in shape. As used herein, the term "cylindrical" indicates a three-dimensional shape that extends along an axis between two ends and has a substantially uniform circular cross-section transverse to the axis from one end to the other; the surfaces at the two ends are not necessarily planar and may be contoured (e.g., convex, concave, etc.).

According to one embodiment, the first cutting apparatus 810 may include a femtosecond laser. Alternatively, the first cutting apparatus 810 may include a single blade that is operable to move along the axis and cut the donor cornea 50 from the anterior surface to the posterior surface, where the blade has a shape that defines a cross-section transverse to the axis. For instance, the blade may be substantially cylindrical so that the portion 52 of corneal tissue is correspondingly substantially cylindrical in shape. In effect, the blade may punch the donor cornea 50 to produce the portion 52 of corneal tissue. Alternatively, the first cutting apparatus 810 may include more than one blade that can be applied to the donor cornea 50 in any number of steps to produce three-dimensionally the portion 52 of corneal tissue.

The system 800 also includes a second cutting apparatus 820 that can make a series of cuts transverse to the axis. As such, the second cutting apparatus 810 can form a plurality of lenticules 60 from the portion 52 of corneal tissue. The corneal tissue between consecutive cuts by the second cutting apparatus 810 provides a corresponding lenticule 60 to be shaped for a corneal implant. The distance between the consecutive cuts defines a thickness for the corresponding lenticule 60. According to one embodiment, the second cutting apparatus 810 may include an ultraviolet femtosecond laser, which provides the advantages described herein.

The consecutive cuts made by the second cutting apparatus 820 include a first cut made closest to the anterior surface of the donor cornea 50 and a second cut made closest to the posterior surface of the donor cornea 50. Thus, the corresponding lenticule 60 includes a first surface formed by the first cut and a second surface formed by the second cut. In some embodiments, the second cutting apparatus 820, e.g., including an ultraviolet femtosecond laser, may shape at least one of the first surface or the second surface of the corresponding lenticule 60 to form an corneal implant with a desired refractive profile.

The donor cornea 50 may include a plurality of lamellar layers disposed between the anterior surface and the posterior surface, where the lamellar layers are formed by the lamellae in the corneal tissue. The first cutting apparatus 810 may cut the donor cornea 50 transversely through the lamellar layers, while the second cutting apparatus 820 can make cuts along the lamellar layers.

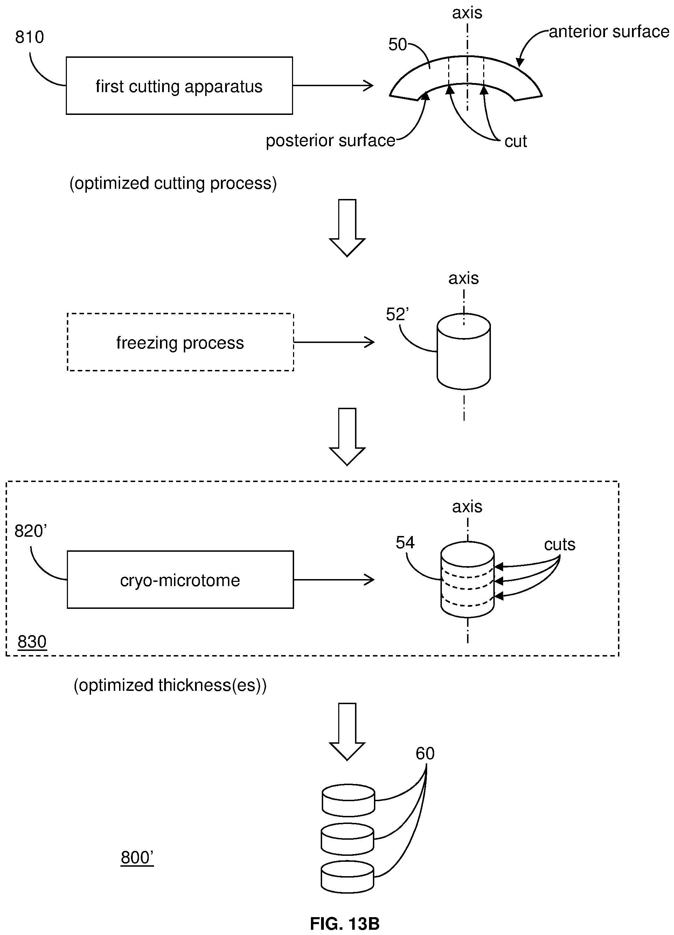

FIG. 13B illustrates an embodiment 800' of the example system 800 for forming a plurality of lenticules 60 from a donor cornea 50. In particular, the cornea 50 is cut to provide a cylinder 52' of corneal tissue with a pre-determined diameter. The corneal tissue cylinder 52' is frozen, and the second cutting apparatus 810 may be a cryo-microtome 820' (freezing microtome, microtome-cryostat, or the like) that slices the frozen corneal tissue cylinder 54 into a plurality of lenticules 60 of desired thickness(es). A cryo-microtome may generally involve the use of a microtome in a temperature-regulated chamber. For instance, a rotary microtome can be adapted to cut in a liquid-nitrogen chamber 830, where the reduced temperature increases the hardness of the corneal tissue cylinder 52' to facilitate the preparation of thin lenticules 60. The temperature of the corneal tissue cylinder 52' of corneal tissue and the second cutting apparatus may be controlled to achieve the desired lenticule thickness(es).

In some cases, a plurality of corneal tissue cylinders 52' may be cut from the donor cornea 50. Taking the desired diameters for the corneal tissue cylinders 52' into account, the locations of the sections of the cornea 50 from which the respective corneal tissue cylinders 52' are cut can be positioned (e.g., mathematically determined) so that as much of the cornea 50 as possible is used to provide the lenticules 60. In other words, waste of the donor cornea 50 is minimized and the greatest possible number of lenticules 60 is obtained from the given donor cornea 50.

Furthermore, to obtain the greatest possible number of lenticules 60 from the given cornea 50, the cryo-microtome 820' may slice the lenticules 60 from the frozen corneal tissue cylinders 54 such that each lenticule 60 has a thickness that is slightly greater than the maximum thickness required for the desired application for the lenticule 60. For instance, the lenticule 60 may be shaped to form an implant with a particular thickness profile to provide a refractive correction of a particular diopter. The thickness of the lenticule 60 is sufficient to achieve this thickness profile while also minimizing waste during the shaping of the implant. In one example, an implant is shaped from the lenticule 60 with a certain central thickness to correct four diopter hyperopia. If another implant is shaped to correct five diopter hyperopia, the corresponding lenticule 60 would have a thickness that is greater by an amount equal to the difference of the four diopter implant and the five diopter implant. Because the cryo-microtome 820' has a cutting resolution down to the micron level, the approach 800' advantageously provides for efficient use of the donor cornea 50 not possible with any other approach.

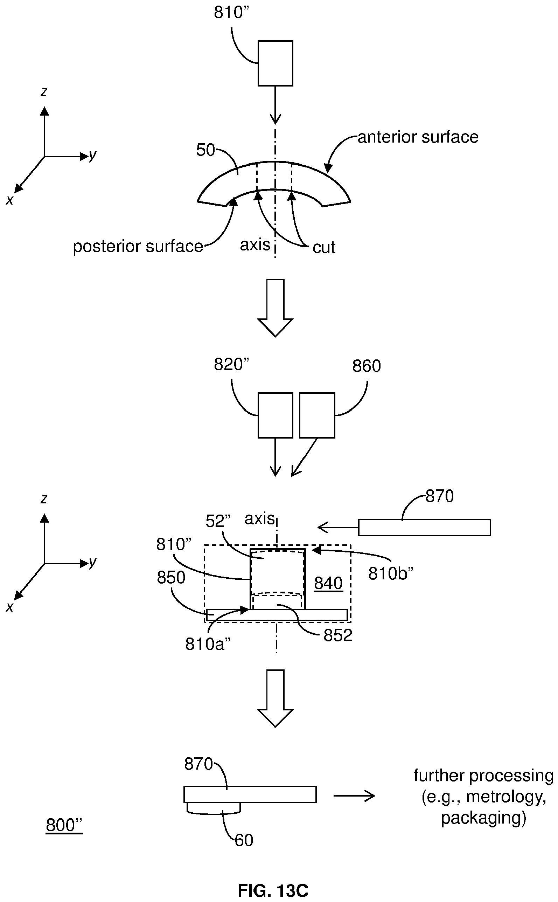

As described above with reference to FIG. 13A, the first cutting apparatus 810 may include a substantially cylindrical blade that cuts the donor cornea 50 so that the portion 52 of corneal tissue is correspondingly substantially cylindrical in shape. FIG. 13C illustrates an example system 800", where the cylindrical blade 810" is configured to produce and hold a cylinder 52" of corneal tissue as the second cutting apparatus 820" makes a series of cuts to the corneal tissue cylinder 52". The second cutting apparatus 820" may be an ultraviolet femtosecond laser with a high numerical aperture (NA), e.g., greater than 0.8.

The system 800" may include a source of hydration fluid 840, and the cylindrical blade 810" can be immersed in the source of hydration fluid 840 while holding the corneal tissue cylinder 52". As such, the hydration fluid 840 keeps the corneal tissue cylinder 52" hydrated while the second cutting apparatus 820" cuts the portion of corneal tissue. The cylindrical blade 810" may include apertures or the like to allow the hydration fluid 840 into cylindrical blade 810" into contact with the corneal tissue cylinder 52". The temperature of the hydration fluid 840 can also be controlled to keep the corneal tissue cylinder 52" at a proper temperature.

The system 800" may additionally include a cutting stage 850. The cylindrical blade 810" is configured to be mounted on the cutting stage 850 as the second cutting apparatus 820" makes the series of cuts to the corneal tissue cylinder 52". The cuts are made at least along the x-y plane as shown in FIG. 13C. A first end 810a" (e.g., bottom) of the cylindrical blade 810" is positioned on, or otherwise engaged by, the cutting stage 850, and the second cutting apparatus 820" is positioned to make the series of cuts at a second opposing end 810b" of (e.g., above) the cylindrical blade 810".

The system 800" may include a guide system 860 that determines the position of the corneal tissue cylinder 52" for the second cutting apparatus 820". For instance, the guide system 840 may be an optical system that provides image or other data to a controller that can determine the position of the corneal tissue cylinder 52" from the data and control the operation of the second cutting apparatus 820".

The cutting stage 830 may include an actuator 852, such as a piezoelectric actuator or similar electromechanical device, that contacts the corneal tissue cylinder 52" at the first end 810a" of the cylindrical blade 810" and moves (e.g., pushes) the corneal tissue cylinder 52" through the cylindrical blade 810" toward the second end 810b" as the second cutting apparatus 820" makes the series of cuts to the corneal tissue cylinder 52".

Consecutive cuts made by the second cutting apparatus 820" include a first cut made closest to the anterior surface of the donor cornea 50 and a second cut made closest to the posterior surface of the donor cornea 50. The consecutive cuts form a corresponding lenticule 60 that includes a first surface formed by the first cut and a second surface formed by the second cut. The second cutting apparatus 820" may also shape at least one of the first surface or the second surface of the corresponding lenticule 60 to form the corneal implant with a desired refractive profile.

For instance, if the second cutting apparatus 820" is an ultraviolet femtosecond laser, the ultraviolet femtosecond laser can shape (e.g., sculpt) the most anterior (e.g., top) surface of the corneal tissue cylinder 52" along the x-, y-, z-axes according to a desired refractive profile. According to one approach, the laser spot of the ultraviolet femtosecond laser may be kept fixed in space and the cutting stage 850 may be operated to move along the x-, y-, z-axes relative to the laser spot. According to another approach, the laser of the ultraviolet femtosecond laser scans the corneal tissue cylinder 52" along the x-, y-, z-axes. The first surface of a lenticule 60 is formed when the most anterior surface is shaped. The ultraviolet femtosecond laser then makes a second cut to form the second surface of the lenticule 60 and release the lenticule 60 from the corneal tissue cylinder 52". The second surface may be planar or contoured (e.g., concave). In general, the lenticule 60 may have any size or shape according to the desired refractive profile. Once the second surface is cut, the actuator 852 can move the corneal tissue cylinder 52" toward the second end 510b" (e.g., upwardly) and push the lenticule 60 out of the cylindrical blade 510".

The system 800" may also include a robotic arm 870 that retrieves each lenticule 60 as it is formed by the second cutting apparatus 810 and pushed out of the cylindrical blade 510" by the actuator 852. The robotic arm 870 may apply a negative pressure to the lenticule 60 to hold the lenticule 60 by suction. The robotic arm 870 may transport the lenticule 60 for further processing, such as measurement and/or packaging.

Although the system 800" may demonstrate how the second cutting apparatus 820 can cut the portion 52 of corneal tissue held by the first cutting apparatus 810. Other approaches are possible. For instance, a cylindrical blade can hold the portion 52 of corneal tissue and a ultraviolet femtosecond laser can make a series of cuts starting from the most posterior surface of the portion 52 of corneal tissue and proceeding toward the most anterior surface (e.g., working from bottom to top). Such an approach may produce meniscus-shaped lenses in particular, but other shapes are possible. When the series of cuts by the ultraviolet femtosecond laser is completed, the portion 52 of corneal tissue can be pushed through the cylindrical blade to produce a "pile" of lenticules 60.

As referenced above, U.S. Patent Application Publication No. 2014/0264980, filed January 10, 2014, and U.S. Patent Application Publication No. 2017/0027754, filed February 28, 2016, further describe the production of lenticules from laminar sheets that are cut from donor cornea.

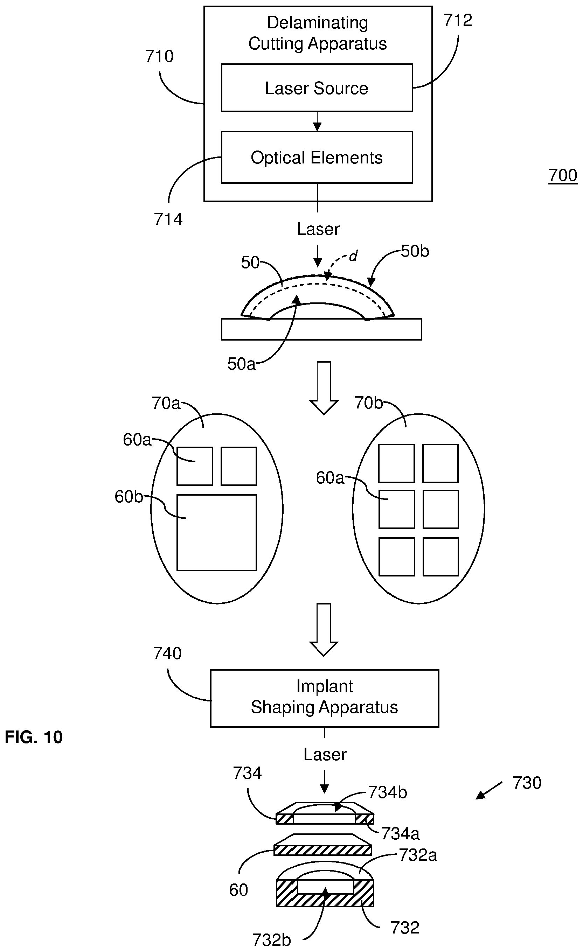

FIG. 10 illustrates an example approach 700 for shaping a lenticule to achieve a desired corneal implant. Laminar sheets may be cut from donor tissue 50 as described above. In particular, a delaminating cutting apparatus 710 includes a light source 712 that generates radiation for dissecting tissue 50a of the donor cornea 50 via photodisruption, photothermal cavitation, laser spallation, etc. The cutting apparatus 710 also includes an optical system 714 that can focus the radiation on the tissue 50a and cut the cornea 50 at a desired depth d below the anterior surface 50b of the cornea 50. In some embodiments, the cutting apparatus 710 may generate a femtosecond laser to cut the cornea 50.

As shown in FIG. 10, lenticules 60 are then cut from the laminar sheets with varying sizes. For instance, from a laminar sheet 70a, two lenticules 60a are cut with a size that can be used to form implants 10a for treating presbyopia, and one lenticule 60b is cut with a size that can be used to form implants 10b for treating hyperopia. If three laminar sheets 70a can be cut from the donor cornea 50, six presbyopia lenticules 60a and three hyperopia lenticules 60b can be obtained from the cornea 50. Additionally or alternatively, from a laminar sheet 70b, six presbyopia lenticules 60a are cut, and with three such laminar sheets 70b, eighteen presbyopia lenticules 60a can be obtained from the cornea 50.

A holding system 730 mechanically positions a lenticule 60 to receive shaping (e.g., ablative) radiation from an implant shaping apparatus 740. The holding system 730 includes a lower structure 732 and an upper structure 734. The lower structure 732 has a round (e.g., circular) upper surface 732a that receives the lenticule 60. The upper structure 734 has a rectangular lower surface 734a that holds (e.g., fixes) the lenticule 60 in position on the lower structure 732. The rectangular shape allows the upper structure 734 to engage the lenticule 60. The width (e.g., diameter) of the upper surface 732a of the lower structure 732 may be substantially equal to an edge length of the lower surface 734a of the upper structure 734. The lower structure 732 includes a round (e.g., circular) aperture 732b and the upper structure 734 includes a corresponding round (e.g., circular) aperture 734b. The apertures 732b, 734b allow the implant shaping apparatus 740 to shape the lenticule 60 between the lower structure 732 and the upper structure 734.

According to some approaches, a lenticule may be prepared and packaged (e.g., by a supplier) for delivery and subsequent reshaping (e.g., by a practitioner) at or near the time of actual implantation into the cornea. As such, the lenticule may provide a more general shape (e.g., a blank) that can be subsequently reshaped into an implant according to any specific shape. As described above, the specific shape may cause a change in refractive power when implanted. In addition, the shape may include desired edge characteristics and other features that allow the structure of the implant to blend or transition smoothly into the surrounding eye structure, for instance, to improve optics and/or promote epithelial growth over the implant.

If a separate supplier packages and delivers a lenticule as a blank to a practitioner, the practitioner may need to know the starting measurements of the lenticule so that the proper amount of tissue can be accurately removed from the lenticule to obtain a precisely shaped corneal implant. In some approaches, the supplier may take the measurements of the lenticule prior to packaging and may provide the measurements to the practitioner. Additionally, the supplier may provide instructions that the practitioner can follow to reshape the lenticule in order to obtain a particular shape for the implant. For instance, the instructions may indicate what tissue should be removed from particular locations of the lenticule. Such instructions are based on the measurements taken of the lenticule.

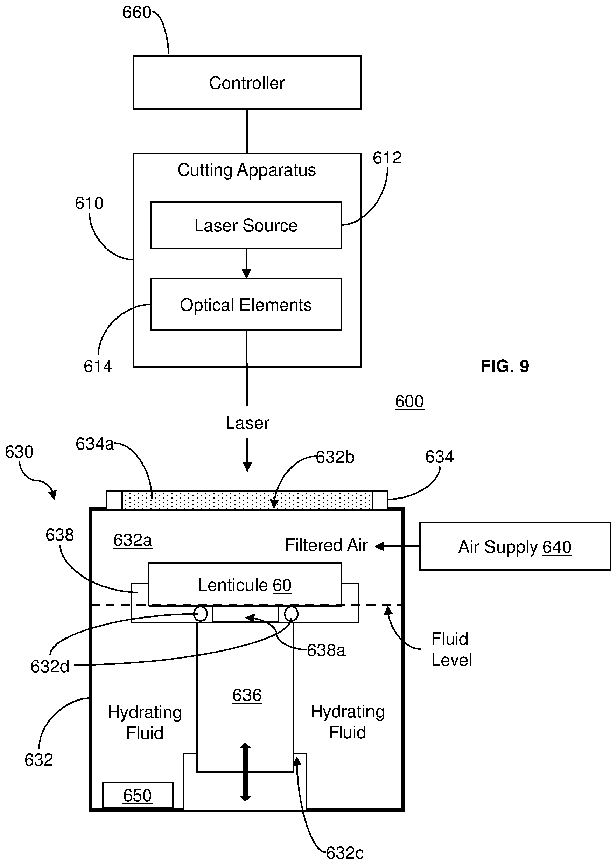

Where a lenticule is delivered as a blank to a practitioner, the practitioner may subsequently employ a reshaping system to reshape the lenticule. FIG. 9 illustrates an example reshaping system 600 that allows a lenticule 60 to be reshaped in a controlled environment. As shown in FIG. 9, the reshaping system 600 includes a lenticule cutting apparatus 610. The cutting apparatus 610 may include a laser source 612 that emits a laser, such as an excimer laser, capable of cutting corneal tissue. The cutting apparatus 610 may also include one or more optical elements 614 that direct the laser from the laser source. Such optical elements 614 may include any combination of lenses, mirrors, filters, beam splitters, etc.

The example reshaping system 600 includes a staging device 630 that stages the lenticule 60 for reshaping by the cutting apparatus 610, while keeping the lenticule 60 in a proper state (e.g., hydration, temperature, etc.). As shown in FIG. 9, the staging device 630 includes a container 632. The container 632 includes a chamber 632a, an upper opening 632b, and a lower opening 632c. The staging device 630 also includes a cover 634 to cover the upper opening 632b via threaded engagement, friction fit, clamps, or the like.

The staging device 630 includes a plunger 636 that passes through the lower opening 632c into the chamber 632a. The plunger 636 includes an upper end 636a and a lower end 636b. The upper end 636a is disposed in the chamber 632a while the lower end 636b is accessible outside the chamber 632a from the bottom of the container 632. Pressure applied against the lower end 636b causes the plunger 636 to move farther through the lower opening 632c and into the chamber 632a. In effect, this pressure raises the upper end 636a within the chamber 632a.

Conversely, the lower end 636b can be pulled away from the container 632 to retract the plunger 636 from the chamber 632a. In effect, this action lowers the upper end 636a within the chamber 632a.

In some embodiments, the plunger 636 can be manually operated. In other embodiments, the plunger 636 may be actuated by a machine, e.g., electro-mechanical device.

The staging device 630 includes a holder 638 that is configured to hold the lenticule 60. The upper end 636a of the plunger 636 is configured to receive the holder 638. According to one approach, a supplier can pack the lenticule 60 in the holder 638 for delivery to the practitioner. As delivered, holder 638 facilitates handling of the lenticule 60. In particular, the lenticule 60 is already properly oriented in the holder 632 (e.g., with the correct surface facing up) for subsequent reshaping with the cutting apparatus 610.

In operation, the cover 634 is removed from the upper opening 632b and the holder 638 with the lenticule 60 is positioned on the upper end 636a of the plunger 636. The chamber 632a is then filled to a predetermined level with hydrating fluid, e.g., balanced salt solution (BSS) or other standardized salt solution, to maintain the lenticule 60 in a hydrated state. The plunger 636 is positioned so that the lenticule 60 is submerged in the hydrating fluid. A liquid-tight seal is provided around the plunger 636 at the lower opening 632c so that liquid can be contained in the chamber 632a without escaping through the lower opening 632c. The cover 634 is then secured over the upper opening 632b of the container 632. Accordingly, the container 634 provides an enclosure in which the lenticule 60 can be reshaped in a controlled environment by the cutting apparatus 610.

The cover 634 includes a window 634a that allows the cutting apparatus 610 to direct a laser into the chamber 632a to cut the lenticule 60 while the holder 638 with the lenticule 60 is disposed on the upper end 636a of the plunger 636. For instance, the window 634d may be formed from quartz that allows transmission of ultraviolet light, e.g., light having a wavelength equal to approximately 193 nm.

In further operation, when the cutting apparatus 610 is aligned with the container 632 to reshape the lenticule 60, pressure can be applied to the lower end 636b of the plunger 636 to raise the holder 638 on the upper end 636a within the chamber 632a. The container 632 also includes one or more stops 632d that stop the plunger 636 from advancing beyond a predetermined distance within the chamber 632a. In particular, when the plunger 636 advances to the stops 632d, the lenticule 60 moves above the predetermined level of hydrating fluid to allow the laser from the cutting apparatus 610 to act on the lenticule 60. The posterior surface of the lenticule 60, however, may remain in contact with the hydrating fluid to maintain hydration. Indeed, the holder 638 may include an aperture 638a to promote contact between the lenticule 60 and the hydrating fluid. As shown in FIG. 9, the plunger 636 abuts the stops 632d and the lenticule 60 is raised above the predetermined fluid level; from this position, the plunger 636 can be retracted to submerge the lenticule 60 in the hydrating fluid.

The staging device 630 also includes an air supply 640 that can deliver filtered air into the chamber 632a. Once the lenticule 60 is above the predetermined level of hydrating fluid, the air supply 640 can blow filtered air toward the lenticule 60 to dry and clean the lenticule 60 sufficiently for the reshaping process. Furthermore, as the cutting apparatus 610 reshapes the lenticule 60, the air supply 640 can continue to blow the filtered air to remove any ablation plume. The air supply 640 can deliver the air toward the lenticule 60 while avoiding direct air flow which may cause dehydration. After the lenticule 60 is reshaped, the plunger 636 can be retracted to draw the lenticule 60 back into the hydrating fluid.

The staging device 630 may also include a heating element 650 that can control the temperature of the hydrating fluid. For instance, the temperature of the hydrating fluid may be raised to 37°C, which maintains the lenticule 60 at physiological temperature and provides humidity within the enclosure of the chamber 632a. For instance, the heating element 650 may be a small resistive coil, e.g., formed from Nichrome, and the temperature may be controlled by a thermistor.

The hydrating fluid may also maintain an isotonic state for the lenticule 60. In vivo, the cornea does not maintain an isotonic thickness, as the corneal endothelial pumps are continually working to dehydrate the cornea slightly. As such, the thickness of the corneal tissue in vivo may be a slightly smaller than what its thickness would be in an isotonic solution. Therefore, when cutting the lenticule 60, the reshaping system 600 may take into account that the implant 10 sculpted from the lenticule 60 may become thinner after it is implanted in the recipient eye.

In general, the reshaping system 600 provides an example of a system for forming a corneal implant, where a receptacle (e.g., container 632) receives a lenticule and maintains a state (e.g., hydration, temperature, etc.) of the lenticule. The receptacle provides a controlled environment for precise and predictable cutting by a cutting apparatus. For instance, the per-pulse cutting rate for a laser may be sensitive to hydration of the lenticule 60, so the lenticule 60 may need to be predictably hydrated for precise and accurate sculpting.

The reshaping system 600 also includes a controller 660, which may be implemented with at least one processor, at least one data storage device, etc., as described further below. The controller 660 is configured to determine a sculpting plan for modifying a first shape of the lenticule 60 and achieving a second shape for the lenticule 60 to produce the implant 10 with the desired refractive profile. The controller 660 can control the cutting apparatus 610 to direct, via the one or more optical elements 614, the laser from the laser source 612 to sculpt the lenticule 60 according to the sculpting plan to produce the implant 10. A monitoring system (not shown) may be employed to guide the laser relative to the position of the lenticule 60. In some cases, the lenticule 60 may be reshaped in relation to predicted corneal biomechanical changes based on the depth of the implant within the cornea. Additionally or alternatively, the lenticule 60 may be reshaped in relation to predicted epithelium changes based on the deformation of the anterior stroma.

As referenced above, U.S. Patent Application Publication No. 2014/0264980, filed January 10, 2014, and U.S. Patent Application Publication No. 2017/0027754, filed February 28, 2016, describe further aspects of reshaping lenticules. Aspects of the present disclosure can be combined with the approaches for reshaping lenticules described in these references.

According to aspects of the present disclosure, some or all of the steps of the above-described and illustrated procedures can be automated or guided under the control of a controller. Generally, the controllers may be implemented as a combination of hardware and software elements. The hardware aspects may include combinations of operatively coupled hardware components including microprocessors, logical circuitry, communication/networking ports, digital filters, memory, or logical circuitry. The controller may be adapted to perform operations specified by a computer-executable code, which may be stored on a computer readable medium.

As described above, the controller may be a programmable processing device, such as an external conventional computer or an on-board field programmable gate array (FPGA) or digital signal processor (DSP), that executes software, or stored instructions. In general, physical processors and/or machines employed by embodiments of the present disclosure for any processing or evaluation may include one or more networked or nonnetworked general purpose computer systems, microprocessors, field programmable gate arrays (FPGA's), digital signal processors (DSP's), micro-controllers, and the like, programmed according to the teachings of the exemplary embodiments of the present disclosure, as is appreciated by those skilled in the computer and software arts. The physical processors and/or machines may be externally networked with the image capture device(s), or may be integrated to reside within the image capture device. Appropriate software can be readily prepared by programmers of ordinary skill based on the teachings of the exemplary embodiments, as is appreciated by those skilled in the software art. In addition, the devices and subsystems of the exemplary embodiments can be implemented by the preparation of application-specific integrated circuits or by interconnecting an appropriate network of conventional component circuits, as is appreciated by those skilled in the electrical art(s). Thus, the exemplary embodiments are not limited to any specific combination of hardware circuitry and/or software.

Stored on any one or on a combination of computer readable media, the exemplary embodiments of the present disclosure may include software for controlling the devices and subsystems of the exemplary embodiments, for driving the devices and subsystems of the exemplary embodiments, for enabling the devices and subsystems of the exemplary embodiments to interact with a human user, and the like. Such software can include, but is not limited to, device drivers, firmware, operating systems, development tools, applications software, and the like. Such computer readable media further can include the computer program product of an embodiment of the present disclosure for performing all or a portion (if processing is distributed) of the processing performed in implementations. Computer code devices of the exemplary embodiments of the present disclosure can include any suitable interpretable or executable code mechanism, including but not limited to scripts, interpretable programs, dynamic link libraries (DLLs), Java classes and applets, complete executable programs, and the like. Moreover, parts of the processing of the exemplary embodiments of the present disclosure can be distributed for better performance, reliability, cost, and the like.

Common forms of computer-readable media may include, for example, a floppy disk, a flexible disk, hard disk, magnetic tape, any other suitable magnetic medium, a CD-ROM, CDRW, DVD, any other suitable optical medium, punch cards, paper tape, optical mark sheets, any other suitable physical medium with patterns of holes or other optically recognizable indicia, a RAM, a PROM, an EPROM, a FLASH-EPROM, any other suitable memory chip or cartridge, a carrier wave or any other suitable medium from which a computer can read.

The present invention is as defined in the appended claims. The embodiments, aspects or examples according to the present description which do not fall within the scope of said claims are provided for illustrative purposes only and do not form part of the present invention.

## Claims

1. A system (800, 800', 800") for forming corneal implants (10), comprising:
a first cutting apparatus (810, 810") configured to cut a donor cornea (50) and form a portion (52, 52', 52", 54) of corneal tissue, wherein the donor cornea (50) includes an anterior surface and a posterior surface, the first cutting apparatus (810) configured to cut the donor cornea (50) along an axis extending between the anterior surface and the posterior surface; and
a second cutting apparatus (820, 820', 820") configured to form a plurality of lenticules (60) from the portion (52, 52', 52", 54) of corneal tissue by making, to the portion (52, 52', 52", 54) of corneal tissue, a series of cuts transverse to the axis, wherein corneal tissue between consecutive cuts by the second cutting apparatus (820, 820', 820") provides a corresponding lenticule (60) to be shaped for a corneal implant (10), a distance between the consecutive cuts defining a thickness for the corresponding lenticule (60), **characterized in that**:
the system further comprises a temperature-regulated chamber (830) configured to maintain the portion (52, 52', 52", 54) of corneal tissue at a low temperature for cutting of the portion of corneal tissue by the second cutting apparatus (820, 820', 820").

2. The system (800, 800') of claim 1, wherein the second cutting apparatus (820) includes a cryo- microtome (820').

3. The system of claim 1, wherein the first cutting apparatus (810) includes a femtosecond laser.

4. The system of claim 1, wherein the second cutting apparatus (820) includes an ultraviolet femtosecond laser (820").

5. The system (800, 800', 800") of claim 1, wherein the consecutive cuts made by the second cutting apparatus (820, 820', 820") include a first cut made closest to the anterior surface of the donor cornea and a second cut made closest to the posterior surface of the donor cornea (50), the corresponding lenticule (60) including a first surface formed by the first cut and a second surface formed by the second cut, and the second cutting apparatus (820, 820', 820") is further configured to shape at least one of the first surface or the second surface of the corresponding lenticule (60) to form the corneal implant (10) with a desired refractive profile.

6. The system (800, 800', 800") of claim 1, wherein the first cutting apparatus (810) includes a blade (810") operable to move along the axis and cut the donor cornea (50) from the anterior surface to the posterior surface, the blade (810") having a shape that defines a cross-section transverse to the axis for the portion (52, 52', 52", 54) of corneal tissue.

7. The system (800, 800', 800") of claim 6, wherein the blade (810") is substantially cylindrical and is operable to provide the portion (52, 52', 52", 54) of corneal tissue with a substantially circular cross-section transverse to the axis.

8. The system (800, 800', 800") of claim 7, wherein the cylindrical blade (810") is configured to hold the portion (52, 52', 52", 54) of corneal tissue as the second cutting apparatus (820, 820', 820") makes the series of cuts to the portion (52, 52', 52", 54) of corneal tissue.

9. The system (800, 800', 800") of claim 8, further comprising a source of hydration fluid (840), wherein the cylindrical blade (810") is configured to be immersed in the source of hydration fluid (840) while holding the portion (52, 52', 52", 54) of corneal tissue, the hydration fluid (840) keeping the portion (52, 52', 52", 54) of corneal tissue hydrated while the second cutting apparatus (820, 820', 820") cuts the portion (52, 52', 52", 54) of corneal tissue.

10. The system (800, 800', 800") of claim 8, further comprising a cutting stage (850), wherein the cylindrical blade (810") is configured to be mounted on the cutting stage (850) as the second cutting apparatus (820, 820', 820") makes the series of cuts to the portion (52, 52', 52", 54) of corneal tissue.

11. The system (800, 800', 800") of claim 10, wherein the second cutting apparatus (820) includes a femtosecond laser, wherein a first end of the cylindrical blade (810") is positioned at the cutting stage (850), and the second cutting apparatus (820) is positioned to make the series of cuts at a second opposing end of the cylindrical blade (810").

12. The system (800, 800', 800") of claim 11, further comprising a guide (860) configured to determine the position of the portion (52, 52', 52", 54) of corneal tissue for the second cutting apparatus (820).

13. The system (800, 800', 800") of claim 11, wherein the consecutive cuts made by the femtosecond laser (820) include a first cut made closest to the anterior surface of the donor cornea and a second cut made closest to the posterior surface of the donor cornea (50), the corresponding lenticule (60) including a first surface formed by the first cut and a second surface formed by the second cut, and the femtosecond laser (820) is configured to shape at least one of the first surface or the second surface of the corresponding lenticule (60) to form the corneal implant (10) with a desired refractive profile.

14. The system (800, 800', 800") of claim 11, wherein the cutting stage (850) includes an actuator (852) configured to contact the portion (52, 52', 52", 54) of corneal tissue at the first end of the cylindrical blade (810") and move the portion (52, 52', 52", 54) of corneal tissue through the cylindrical blade (810') toward the second end as the second cutting apparatus (820) makes the series of cuts to the portion (52, 52', 52", 54) of the corneal tissue.

15. The system (800, 800', 800") of claim 13, further comprising a robotic arm (870) configured to retrieve each corresponding lenticule (60) as the second cutting apparatus (820) makes the consecutive cuts and the actuator (852) moves the portion (52, 52', 52", 54) of corneal tissue through the cylindrical blade (810").

## Patentansprüche

1. System (800, 800', 800") zum Ausbilden von Hornhautimplantaten (10), umfassend:
eine erste Schneidvorrichtung (810, 810"), die ausgelegt ist, um eine Spenderhornhaut (50) zu schneiden und einen Abschnitt (52, 52', 52", 54) von Hornhautgewebe auszubilden, wobei die Spenderhornhaut (50) eine vordere Oberfläche und eine hintere Oberfläche aufweist, wobei die erste Schneidvorrichtung (810) ausgelegt ist, um die Spenderhornhaut (50) entlang einer Achse zu schneiden, die sich zwischen der vorderen Oberfläche und der hinteren Oberfläche erstreckt; und
eine zweite Schneidvorrichtung (820, 820', 820"), die ausgelegt ist, um eine Vielzahl von Linsen (60) aus dem Abschnitt (52, 52', 52", 54) von Hornhautgewebe auszubilden, indem in dem Abschnitt (52, 52', 52", 54) von Hornhautgewebe eine Reihe von Schnitten quer zur Achse vorgenommen werden, wobei das Hornhautgewebe zwischen aufeinanderfolgenden Schnitten durch die zweite Schneidvorrichtung (820, 820', 820") eine entsprechende Linse (60) bereitstellt, die für ein Hornhautimplantat (10) zu formen ist, wobei ein Abstand zwischen den aufeinanderfolgenden Schnitten eine Dicke für die entsprechende Linse (60) definiert, **dadurch gekennzeichnet, dass**:
das System weiters eine temperaturgesteuerte Kammer (830) umfasst, die ausgelegt ist, um den Abschnitt (52, 52', 52", 54) von Hornhautgewebe zum Schneiden des Abschnitts von Hornhautgewebe durch die zweite Schneidvorrichtung (820, 820', 820") auf einer niedrigen Temperatur zu halten.

2. System (800, 800') nach Anspruch 1, wobei die zweite Schneidvorrichtung (820) ein Kryomikrotom (820') umfasst.

3. System nach Anspruch 1, wobei die erste Schneidvorrichtung (810) einen Femtosekunden-Laser umfasst.

4. System nach Anspruch 1, wobei die zweite Schneidvorrichtung (820) einen UV-Femtosekunden-Laser (820") umfasst.

5. System (800, 800', 800") nach Anspruch 1, wobei die aufeinanderfolgenden Schnitte, die durch die zweite Schneidvorrichtung (820, 820', 820") vorgenommen wurden, einen ersten Schnitt, der am nächsten zu der vorderen Oberfläche der Spenderhornhaut vorgenommen wurde, und einen zweiten Schnitt umfassen, der am nächsten zu der hinteren Oberfläche der Spenderhornhaut (50) vorgenommen wurde, wobei die entsprechende Linse (60) eine erste Oberfläche, die durch den ersten Schnitt ausgebildet ist, und eine zweite Oberfläche umfasst, die durch den zweiten Schnitt ausgebildet ist, und wobei die zweite Schneidvorrichtung (820, 820', 820") ferner ausgelegt ist, um zumindest eine aus der ersten Oberfläche oder der zweiten Oberfläche der entsprechenden Linse (60) zu formen, um das Hornhautimplantat (10) mit einem gewünschten Brechungsindexprofil auszubilden.

6. System (800, 800', 800") nach Anspruch 1, wobei die erste Schneidvorrichtung (810) eine Klinge (810") umfasst, die betreibbar ist, um sich entlang der Achse zu bewegen und die Spenderhornhaut (50) von der vorderen Oberfläche zur hinteren Oberfläche zu schneiden, wobei die Klinge (810") eine Form aufweist, die einen Querschnitt quer zur Achse für den Abschnitt (52, 52', 52", 54) von Hornhautgewebe definiert.

7. System (800, 800', 800") nach Anspruch 6, wobei die Klinge (810") im Wesentlichen zylindrisch und betreibbar ist, um den Abschnitt (52, 52', 52", 54) von Hornhautgewebe mit einem im Wesentlichen kreisförmigen Querschnitt quer zur Achse bereitzustellen.

8. System (800, 800', 800") nach Anspruch 7, wobei die zylindrische Klinge (810") ausgelegt ist, um den Abschnitt (52, 52', 52", 54) von Hornhautgewebe zu halten, während die zweite Schneidvorrichtung (820, 820', 820") die Reihe von Schnitten in dem Abschnitt (52, 52', 52", 54) von Hornhautgewebe vornimmt.

9. System (800, 800', 800") nach Anspruch 8, das ferner eine Quelle eines Hydrierungsfluids (840) umfasst, wobei die zylindrische Klinge (810") ausgelegt ist, um in die Quelle von Hydrierungsfluid (840) eingetaucht zu werden, während der Abschnitt (52, 52', 52", 54) von Hornhautgewebe gehalten wird, wobei das Hydrierungsfluid (840) den Abschnitt (52, 52', 52", 54) von Hornhautgewebe hydriert hält, während die zweite Schneidvorrichtung (820, 820', 820") den Abschnitt (52, 52', 52", 54) von Hornhautgewebe schneidet.

10. System (800, 800', 800") nach Anspruch 8, das ferner eine Schneidplattform (850) umfasst, wobei die zylindrische Klinge (810") ausgelegt ist, um auf der Schneidplattform (850) montiert zu werden, während die zweite Schneidvorrichtung (820, 820', 820") die Reihe von Schnitten in dem Abschnitt (52, 52', 52", 54) von Hornhautgewebe vornimmt.

11. System (800, 800', 800") nach Anspruch 10, wobei die zweite Schneidvorrichtung (820) einen Femtosekunden-Laser umfasst, wobei ein erstes Ende der zylindrischen Klinge (810") an der Schneidplattform (850) angeordnet ist, und wobei die zweite Schneidvorrichtung (820) angeordnet ist, um die Reihe von Schnitten an einem zweiten gegenüberliegenden Ende der zylindrischen Klinge (810") vorzunehmen.

12. System (800, 800', 800") nach Anspruch 11, das ferner eine Führung (860) umfasst, die ausgelegt ist, um die Position des Abschnitts (52, 52', 52", 54) von Hornhautgewebe für die zweite Schneidvorrichtung (820) zu bestimmen.

13. System (800, 800', 800") nach Anspruch 11, wobei die aufeinanderfolgenden Schnitte, die durch den Femtosekunden-Laser (820) vorgenommen werden, einen ersten Schnitt, der am nächsten zu der vorderen Oberfläche der Spenderhornhaut vorgenommen wird, und einen zweiten Schnitt umfasst, der am nächsten zu der hinteren Oberfläche der Spenderhornhaut (50) vorgenommen wird, wobei die entsprechende Linse (60) eine erste Oberfläche, die durch den ersten Schnitt ausgebildet wird, und eine zweite Oberfläche aufweist, die durch den zweiten Schnitt ausgebildet wird, und wobei der Femtosekunden-Laser (820) ausgelegt ist, um zumindest eine aus der ersten Oberfläche und der zweiten Oberfläche der entsprechenden Linse (60) zu formen, um das Hornhautimplantat (10) mit einem gewünschten Brechungsindexprofil auszubilden.

14. System (800, 800', 800") nach Anspruch 11, wobei die Schneidplattform (850) ein Betätigungselement (852) umfasst, das ausgelegt ist, um den Abschnitt (52, 52', 52", 54) von Hornhautgewebe am ersten Ende der zylindrischen Klinge (810") zu berühren und den Abschnitt (52, 52', 52", 54) von Hornhautgewebe durch die zylindrische Klinge (810') in Richtung des zweiten Endes zu bewegen, während die zweite Schneidvorrichtung (820) die Reihe von Schnitten in dem Abschnitt (52, 52', 52", 54) von Hornhautgewebe vornimmt.

15. System (800, 800', 800") nach Anspruch 13, das ferner einen Roboterarm (870) umfasst, der ausgelegt ist, um jede entsprechende Linse (60) zu entnehmen, während die zweite Schneidvorrichtung (820) die aufeinanderfolgenden Schnitte vornimmt und das Betätigungselement (852) den Abschnitt (52, 52', 52", 54) von Hornhautgewebe durch die zylindrische Klinge (810") bewegt.

## Revendications

1. Système (800, 800', 800") pour former des implants cornéens (10), comprenant :
un premier appareil de coupe (810, 810") configuré pour couper une cornée de donneur (50) et former une partie (52, 52', 52", 54) de tissu cornéen, dans lequel la cornée de donneur (50) comprend une surface antérieure et une surface postérieure, le premier appareil de coupe (810) étant configuré pour couper la cornée de donneur (50) le long d'un axe s'étendant entre la surface antérieure et la surface postérieure ; et
un second appareil de coupe (820, 820', 820") configuré pour former une pluralité de lenticules (60) à partir de la partie (52, 52', 52", 54) de tissu cornéen en réalisant, sur la partie (52, 52', 52", 54) de tissu cornéen, une série de coupes transversales à l'axe, dans lequel le tissu cornéen entre des coupes consécutives par le second appareil de coupe (820, 820', 820") fournit une lenticule correspondante (60) à mettre en forme pour un implant cornéen (10), une distance entre les coupes consécutives définissant une épaisseur pour la lenticule correspondante (60), **caractérisé en ce que** :
le système comprend en outre une chambre régulée en température (830) configurée pour maintenir la partie (52, 52', 52", 54) de tissu cornéen à une température basse afin de couper la partie de tissu cornéen par le second appareil de coupe (820, 820', 820").

2. Système (800, 800') selon la revendication 1, dans lequel le second appareil de coupe (820) inclut un cryo-microtome (820').

3. Système selon la revendication 1, dans lequel le premier appareil de coupe (810) inclut un laser femtoseconde.

4. Système selon la revendication 1, dans lequel le second appareil de découpe (820) comprend un laser femtoseconde ultraviolet (820").

5. Système (800, 800', 800") selon la revendication 1, dans lequel les coupes consécutives effectuées par le second appareil de coupe (820, 820', 820") incluent une première coupe effectuée la plus proche de la surface antérieure de la cornée de donneur et une seconde coupe effectuée la plus proche de la surface postérieure de la cornée de donneur (50), la lenticule correspondante (60) incluant une première surface formée par la première coupe et une seconde surface formée par la seconde coupe, et le second appareil de coupe (820, 820', 820") est en outre configuré pour mettre en forme au moins l'une de la première surface ou de la seconde surface de la lenticule correspondante (60) pour former l'implant cornéen (10) avec un profil réfractif souhaité.

6. Système (800, 800', 800") selon la revendication 1, dans lequel le premier appareil de coupe (810) inclut une lame (810") pouvant fonctionner pour se déplacer le long de l'axe et couper la cornée de donneur (50) de la surface antérieure à la surface postérieure, la lame (810") présentant une forme qui définit une section transversale à l'axe pour la partie (52, 52', 52", 54) de tissu cornéen.

7. Système (800, 800', 800") selon la revendication 6, dans lequel la lame (810") est sensiblement cylindrique et peut fonctionner pour fournir à la partie (52, 52', 52", 54) de tissu cornéen une section transversale sensiblement circulaire à l'axe.

8. Système (800, 800', 800") selon la revendication 7, dans lequel la lame cylindrique (810") est configurée pour maintenir la partie (52, 52', 52", 54) de tissu cornéen lorsque le second appareil de coupe (820, 820', 820") effectue la série de coupes sur la partie (52, 52', 52", 54) de tissu cornéen.

9. Système (800, 800', 800") selon la revendication 8, comprenant en outre une source de fluide d'hydratation (840), dans lequel la lame cylindrique (810") est configurée pour être immergée dans la source de fluide d'hydratation (840) tout en maintenant la partie (52, 52', 52", 54) de tissu cornéen, le fluide d'hydratation (840) maintenant la partie (52, 52', 52", 54) de tissu cornéen hydratée tandis que le second appareil de coupe (820, 820', 820") coupe la partie (52, 52', 52", 54) de tissu cornéen.

10. Système (800, 800', 800") selon la revendication 8, comprenant en outre une platine de coupe (850), dans lequel la lame cylindrique (810") est configurée pour être montée sur la platine de coupe (850) lorsque le second appareil de coupe (820, 820', 820") effectue la série de coupes sur la partie (52, 52', 52", 54) de tissu cornéen.

11. Système (800, 800', 800") selon la revendication 10, dans lequel le second appareil de coupe (820) inclut un laser femtoseconde, dans lequel une première extrémité de la lame cylindrique (810") est positionnée au niveau de la platine de coupe (850), et le second appareil de coupe (820) est positionné pour effectuer la série de coupes au niveau d'une seconde extrémité opposée de la lame cylindrique (810").

12. Système (800, 800', 800") selon la revendication 11, comprenant en outre un guide (860) configuré pour déterminer la position de la partie (52, 52', 52", 54) de tissu cornéen pour le second appareil de coupe (820) .

13. Système (800, 800', 800") selon la revendication 11, dans lequel les coupes consécutives effectuées par le laser femtoseconde (820) incluent une première coupe effectuée la plus proche de la surface antérieure de la cornée de donneur et une seconde coupe effectuée la plus proche de la surface postérieure de la cornée de donneur (50), la lenticule correspondante (60) incluant une première surface formée par la première coupe et une seconde surface formée par la seconde coupe, et le laser femtoseconde (820) est configuré pour former au moins une parmi la première surface ou la seconde surface de la lenticule correspondante (60) pour former l'implant cornéen (10) avec un profil de réfraction souhaité.

14. Système (800, 800', 800") selon la revendication 11, dans lequel la platine de coupe (850) inclut un actionneur (852) configuré pour venir en contact avec la partie (52, 52', 52", 54) de tissu cornéen au niveau de la première extrémité de la lame cylindrique (810") et déplacer la partie (52, 52 ', 52", 54) de tissu cornéen à travers la lame cylindrique (810') vers la seconde extrémité lorsque le second appareil de coupe (820) effectue la série de coupes sur la partie (52, 52', 52", 54) du tissu cornéen.

15. Système (800, 800', 800") selon la revendication 13, comprenant en outre un bras robotisé (870) configuré pour récupérer chaque lenticule correspondante (60) lorsque le second appareil de coupe (820) effectue les coupes consécutives et l'actionneur (852) déplace la partie (52, 52', 52", 54) de tissu cornéen à travers la lame cylindrique (810").
